# DEMANDE DE BREVET EUROPEEN

(11) **EP 0 984 007 A1**
(43) Date de publication de la demande: **08.03.2000**
(21) Numéro de dépôt: 99401937.0
(22) Date de dépôt: 28.07.1999
(51) Int. Cl.: C07D 233/54, C07D 213/20, A61K 7/13

(54) **Nouvelles orthophénylènediamines cationiques, leur utilisation pour la teinture d'oxydation des fibres kératiniques, compositions tinctoriales et procédés de teinture**

(30) Priorité: 02.09.1998 FR 9810978
(71) Demandeur: L'OREAL, 75008 Paris (FR)
(72) Inventeur: Genet, Alain, 93600 Aulnay-sous-Bois (FR); Lagrange, Alain, 77700 Coupvray (FR)
(74) Mandataire: Goulard, Sophie

(57) **Abrégé**

L'invention a pour objet de nouvelles orthophénylènediamines monobenzéniques comportant au moins un groupement cationique Z, Z étant choisi parmi des chaînes aliphatiques comportant au moins un cycle insaturé quaternisé, leur utilisation pour la teinture d'oxydation des fibres kératiniques, les compositions tinctoriales les contenant, ainsi que les procédés de teinture d'oxydation les mettant en oeuvre.

## Description

L'invention a pour objet de nouvelles orthophénylènediamines monobenzéniques comportant au moins un groupement cationique Z, Z étant choisi parmi des chaînes aliphatiques comportant au moins un cycle insaturé quaternisé, leur utilisation pour la teinture d'oxydation des fibres kératiniques, les compositions tinctoriales les contenant, ainsi que les procédés de teinture d'oxydation les mettant en oeuvre.

ll est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir, de façon totalement inattendue et surprenante, qu'une nouvelle famille d'orthophénylènediamines monobenzéniques de formule (I) ci-après définie, comportant au moins un groupement cationique Z, Z étant choisi parmi des chaînes aliphatiques comportant au moins un cycle insaturé quaternisé, conviennent pour une utilisation comme base d'oxydation ou comme coupleur ou comme composé auto-oxydable pour la coloration d'oxydation, mais en outre qu'ils permettent d'obtenir des compositions tinctoriales conduisant à des colorations puissantes, dans une très large palette de nuances et présentant d'excellentes propriétés de résistance aux différents traitements que peuvent subir les fibres kératiniques. Enfin, ces composés s'avèrent être aisément synthétisables.

Ces découvertes sont à la base de la présente invention.

L'invention a donc pour premier objet de nouvelles orthophénylènediamines de formule (I) suivante et leurs sels d'addition avec un acide : 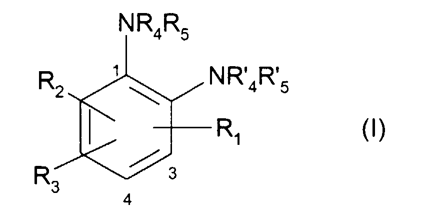 dans laquelle :
- R₁, R₂, R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z dans lequel le bras de liaison D comporte une fonction cétone directement rattachée à l'atome d'azote dudit groupe amino ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
- R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et le groupement Z ;
- R₄, R'₄, R₅ et R'₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
- Z est choisi parmi les groupements cationiques insaturés de formules (II) et (III) suivantes, et les groupements cationiques saturés de formule (lV) suivante : 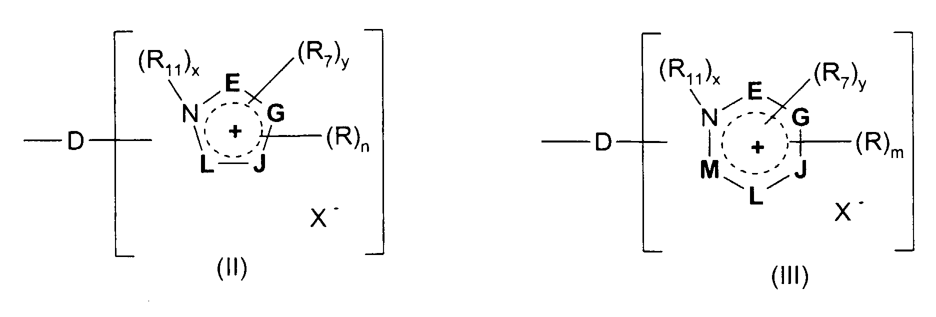 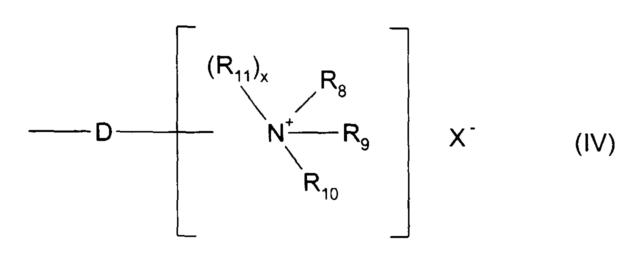 dans lesquelles :
   - D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
   - les sommets E, G, J, L et M, identiques ou différents, représentent un atome de carbone, d'oxygène, de soufre ou d'azote ;
   - n est un nombre entier compris entre 0 et 4 inclusivement ;
   - m est un nombre entier compris entre 0 et 5 inclusivement ;
   - les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
   - R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
   - R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
   l'un des radicaux R₈, R₉ et R₁₀ peut également représenter un second groupement Z identique ou différent du premier groupement Z ;
   - R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆; un radical N-alkyl(C₁-C₆)sulfonamidoalkyle en C₁-C₆ ;
   - x et y sont des nombres entiers égaux à 0 ou 1 ; avec les conditions suivantes :
      - dans les groupements cationiques insaturés de formule (ll) :
         - lorsque x = 0, le bras de liaison D est rattaché à l'atome d'azote,
         - lorsque x = 1, le bras de liaison D est rattaché à l'un des sommets E, G, J ou L,
         - y ne peut prendre la valeur 1 que:
            1) lorsque les sommets E, G, J et L représentent simultanément un atome de carbone, et que le radical R₇ est porté par l'atome d'azote du cycle insaturé ; ou bien
            2) lorsqu'au moins un des sommets E, G, J et L représente un atome d'azote sur lequel le radical R₇ est fixé ;
      - dans les groupements cationiques insaturés de formule (lll) :
         - lorsque x = 0, le bras de liaison D est rattaché à l'atome d'azote,
         - lorsque x = 1, le bras de liaison D est rattaché à l'un des sommets E, G, J, L ou M,
         - y ne peut prendre la valeur 1 que lorsqu'au moins un des sommets E, G, J, L et M représente un atome divalent, et que le radical R₇ est porté par l'atome d'azote du cycle insaturé ;
         - dans les groupements cationiques de formule (lV) :
            - lorsque x = 0, alors le bras de liaison est rattaché à l'atome d'azote portant les radicaux R₈ à R₁₀,
            - lorsque x = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
   - X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
   étant entendu que :
   - le nombre de groupements cationiques insaturés Z de formule (II) ou (III) est au moins égal à 1 ;
   - lorsque R₄ et/ou R₅ et/ou R'₄ et/ou R'₅ représente un groupement Z dans lequel le bras de liaison D représente une chaîne alkyle comportant une fonction cétone, alors ladite fonction cétone n'est pas directement rattachée à l'atome d'azote du groupement -NR₄R₅ ou -NR'₄R'₅.

Comme indiqué précédemment, ces nouvelles orthophénylènediamines de formule (I) sont des composés pouvant être utilisés pour la coloration d'oxydation des fibres kératiniques et présentent l'avantage de se comporter à la fois comme une base d'oxydation et comme un coupleur, ou encore comme des composés auto-oxydables c'est à dire capables de conduire à une coloration des fibres kératiniques sans utiliser d'autre agent oxydant que l'oxygène de l'air. Les compositions de teinture d'oxydation renfermant ces nouvelles orthophénylènediamines de formule (I) conforme à l'invention conduisent à des colorations puissantes dans une très large palette de nuances. Les colorations obtenues en mettant en oeuvre ces orthophénylènediamines de formule (I) présentent de plus d'excellentes propriétés de résistance vis à vis de l'action des différents agents extérieurs (lumière, intempéries, lavage, ondulation permanente, transpiration, frottements).

Dans la formule (I) ci-dessus les radicaux alkyle et alcoxy peuvent être linéaires ou ramifiés.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les composés de formule (I) ci-dessus, on peut notamment citer :
- le monochlorure de 3-[3-(2-amino-phénylamino)-propyl]-1-méthyl-3H-imidazol-1-ium ;
- le monochlorure de 3-[2-(2-amino-phénylamino)-éthyl]-1-méthyl-3H-imidazol-1-ium ;
- le dichlorure de 4-[2-(1-méthyl-3H-imidazol-1-ium)-éthoxy]-N2-[2-(1-méthyl-3H-imidazol-1-ium)-éthyl]-benzène-1,2-diamine ;
- le monochlorure de 3-[2-(2-amino-4-méthyl-phénylamino)-éthyl]-1-éthyl-3H-imidazol-1-ium ;
- le dichlorure de 3-[3-(2-amino-phénylamino)-propyl]-1-(3-triméthyl-ammonium-2-hydroxy-propyl)-3H-imidazol-1-ium ;
- le monobromure de 3-[3-(2-amino-phénylamino)-propyl]-1-(2-hydroxy-éthyl)-3H-imidazol-1-ium
- le monochlorure de 3-{[2-(2-amino-phénylamino)-éthylcarbamoyl]-méthyl}-1-méthyl-3H-imidazol-1-ium ;
- le monochlorure de 1-[2-(2-amino-4-chloro-phénylamino)-éthyl]-pyridinium ;
- le monochlorure de 3-[2-(2-amino-5-méthoxy-phénylamino)-éthyl]-1-méthyl-3H-imidazol-1-ium ;
- le monochlorure de 3-[2-(2-amino-5-méthylsulfanyl-phénylamino)-éthyl]-1-méthyl-3H-imidazol-1-ium ;
et leurs sels d'addition avec un acide.

Les composés de formule (I) conformes à l'invention peuvent être facilement obtenus, selon des méthodes bien connues de l'état de la technique par exemple par réduction des composés nitrés cationiques correspondants (ortho-nitranilines cationiques).

Cette étape de réduction (obtention d'une amine aromatique primaire) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par la "déprotection" (en général en milieu acide) de la fonction amine.

Lorsque la synthèse est terminée, les composés de formule (I) conformes à l'invention peuvent, le cas échéant, être récupérés par des méthodes bien connues de l'état de la technique telles que la cristallisation ou la distillation.

Un autre objet de l'invention est l'utilisation des composés de formule (I) conformes à l'invention à titre de base d'oxydation, de coupleur ou bien encore à titre de colorant auto-oxydable pour la teinture d'oxydation des fibres kératiniques, et en particulier des fibres kératiniques humaines telles que les cheveux.

Les composés de formule (I) conforme à l'invention sont de préférence utilisés à titre de coupleur pour la teinture d'oxydation des fibres kératiniques.

L'invention a également pour objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) conforme à l'invention.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Selon une forme de réalisation préférée de l'invention, la composition tinctoriale renferme en outre une ou plusieurs bases d'oxydation qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre de l'invention (composés de formule (I), bases d'oxydation et coupleurs) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. ll peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 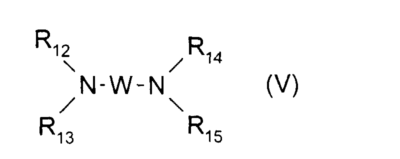 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates et les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. ll est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant présenter un caractère limitatif.

### EXEMPLE DE PREPARATION

### PREPARATION DU MONOCHLORURE DE 3-[3-(2-AMINO-PHENYLAMINO)PROPYL]-1-METHYL-3H-IMIDAZOL-1-IUM, CHLORHYDRATE

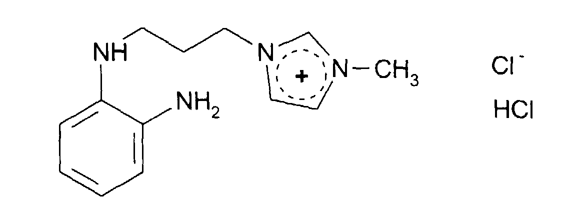

a) Synthèse de la (3-imidazol-1-yl-propyl)-(2-nitro-phényl)-amine
On a chauffé au bain-marie bouillant un mélange de 187,8 g (1,5 moles) de 3-imidazol-1-yl-propylamine et 82,8 g (0,6 mole) de carbonate de potassium dans 280 ml d'eau.
On a ajouté goutte à goutte 141,1 g (1 mole) de 1-fluoro-2-nitro-benzène en 50 minutes et maintenu à une température de 90-95°C pendant 2 heures. On a refroidi dans un bain de glace, essoré le précipité cristallisé, lavé à l'eau et recristallisé de l'isopropanol au reflux.
On a obtenu 109,3 g de cristaux jaune orangé fondant à 80°C (Kofler) et dont l'analyse élémentaire calculée pour C₁₂H₁₄N₄O₂ était :

| % | C | H | N | O |
|---|---|---|---|---|
| Calculé | 58,53 | 5,73 | 22,75 | 12,99 |
| Trouvé | 58,40 | 5,78 | 22,54 | 13,07 |

b) Quaternisation de la (3-imidazol-1-yl-propyl)-(2-nitro-phényl)-amine
On a fait la suspension de 61,5 g (0,25 mole) de (3-imidazol-1-yl-propyl)-(2-nitrophenyl)-amine préparée ci-dessus à l'étape précédente et de 26,0 ml (0,27 mole) de diméthylsulfate dans 800 ml d'acétate d'éthyle et laissé 3 heures à 50°C, sous agitation.
Le précipité huileux a été décanté, lavé plusieurs fois dans l'acétate d'éthyle, et séché à 40°C sous vide.
On a obtenu 81,3 g d'une huile jaune dont l'analyse élémentaire calculée pour C₁₄H₂₀N₄O₆S + ½ H₂O était :

| % | C | H | N | O | S |
|---|---|---|---|---|---|
| Calculé | 44,09 | 5,55 | 14,69 | 27,22 | 8,41 |
| Trouvé | 44,80 | 5,56 | 14,55 | 27,23 | 8,29 |

c) Réduction
Dans un hydrogénateur on a placé 75,0 g (0,196 mole) du composé obtenu ci-dessus à l'étape précédente, 12 g de palladium à 5% sur charbon (contenant 50% d'eau), 300 ml d'éthanol 96°et 300 ml d'eau.
La réduction s'est faite en une ½ heure sous une pression d'hydrogène d'environ 8 bars et à une température qui a progressivement été portée à 75°C. Après filtration du catalyseur sous azote, on a coulé sur 100 ml d'acide chlorhydrique à 36% et évaporé le filtrat à sec sous pression réduite.
Le composé a été repris plusieurs fois dans l'éthanol absolu chlorhydrique environ 5N. Après recristallisation d'un mélange éthanol/eau au reflux et séchage à 40°C sous vide et sur potasse, on a obtenu 23,0 g de cristaux légèrement violet de monochlorure de 3-[3-(2-amino-phénylamino)-propyl]-1-méthyl-3H-imidazol-1-ium, chlorhydrate qui ont fondu avec décomposition à 202-204°C (Kofler) et dont l'analyse élémentaire calculée pour C₁₃H₂₀N₄Cl₂ était :

| % | C | H | N | Cl |
|---|---|---|---|---|
| Calculé | 51,49 | 6,65 | 18,48 | 23,38 |
| Trouvé | 51,22 | 6,71 | 18,37 | 23,29 |

### EXEMPLES D'APPLICATION

### EXEMPLES 1 à 4 DE TEINTURE EN MILIEU ALCALIN

On a préparé les compositions tinctoriales conformes à l'invention suivantes (teneurs en grammes) :

| **EXEMPLE** | **1** | **2** | **3** | **4** |
|---|---|---|---|---|
| Monochlorure de 3-[3-(2-aminophénylamino)-propyl]-1 -méthyl-3H-imidazol-1-ium, chlorhydrate (composé de formule (l)) | 0,909 | 0,909 | 0,909 | 0,909 |
| 4,5-diamino 1-éthyl 3-méthyl pyrazole 2HCl (base d'oxydation) | 0,639 | - | - | - |
| Méta-aminophénol (coupleur) | - | 0,327 | - | - |
| Dichlorhydrate de 2,4-diamino phénoxyéthanol (coupleur) | - | - | 0,723 | - |
| Support de teinture commun n°1 | (*) | (*) | (*) | (*) |
| Eau déminéralisée q.s.p. | 100 g | 100 g | 100 g | 100 g |

| | | | | |
|---|---|---|---|---|
| (*) Support de teinture commun n°1 : - Alcool éthylique à 96° 18 g - Métabisulfite de sodium en solution aqueuse à 35% 0,68 g - Sel pentasodique de l'acide diéthylènetriaminopentacétique 1,1 g - Ammoniaque à 20% 10,0 g | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Blond doré mat |
| **2** | 10 ± 0,2 | Blond mat |
| **3** | 10 ± 0,2 | Vert rabattu |
| **4** | 10 ± 0,2 | Blond doré cuivré |

### EXEMPLES 5 à 8 DE TEINTURE EN MILIEU NEUTRE

On a préparé les compositions tinctoriales conformes à l'invention suivantes (teneurs en grammes) :

| **EXEMPLE** | **5** | **6** | **7** | **8** |
|---|---|---|---|---|
| Monochlorure de 3-[3-(2-aminophénylamino)-propyl]-1-méthyl-3H-imidazol-1-ium, chlorhydrate (composé de formule (l)) | 0,909 | 0,909 | 0,909 | 0,909 |
| Paratoluylènediamine, 2HCl (base d'oxydation) | 0,585 | - | - | - |
| 1,3-dihydroxy benzène (coupleur) | - | 0,33 | - | - |
| 3,7-diaminopyrazolopyrimidine, 2HCl (base d'oxydation) | | - | 0,666 | - |
| 4-hydroxy indole (coupleur) | - | - | - | 0,399 |
| Support de teinture commun n°2 | (**) | (**) | (**) | (**) |
| Eau déminéralisée q.s.p. | 100 g | 100 g | 100 g | 100 g |

| | | | | |
|---|---|---|---|---|
| (**) Support de teinture commun n°2 : - Ethanol à 96° 18 g - Tampon K₂HPO₄ /KH₂PO₄ (1,5 M / 1 M) 10 g - Métabisulfite de sodium 0,68 g - Sel pentasodique de l'acide diéthylènetriaminopentacétique 1,1 g | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **5** | 5,7 ± 0,2 | Châtain foncé cendré mat |
| **6** | 5,7 ± 0,2 | Blond clair doré cuivré |
| **7** | 5,7 ± 0,2 | Vert puissant |
| **8** | 5,7 ± 0,2 | Blond clair doré |

### EXEMPLES 9 à 12 DE TEINTURE EN MILIEU ALCALIN

On a préparé les compositions tinctoriales conformes à l'invention suivantes (teneurs en grammes) :

| **EXEMPLE** | **9** | **10** | **11** | **12** |
|---|---|---|---|---|
| Monochlorure de 3-[3-(2-aminophénylamino)-propyl]-1-méthyl-3H-imidazol-1-ium, chlorhydrate (composé de formule (l)) | 0,909 | 0,909 | 0,909 | 0,909 |
| 3,7-diaminopyrazolopyrimidine, 2HCl (base d'oxydation) | 0,666 | - | - | - |
| 1,3-dihydroxy benzène (coupleur) | - | 0,33 | - | - |
| 4-hydroxy indole (coupleur) | - | - | 0,399 | - |
| Paratoluylènediamine, 2HCl (base d'oxydation) | - | - | - | 0,585 |
| Support de teinture commun n°1 | (*) | (*) | (*) | (*) |
| Eau déminéralisée q.s.p. | 100 g | 100 g | 100 g | 100 g |

| | | | | |
|---|---|---|---|---|
| (*) Support de teinture commun n°1 : II est identique à celui utilisé pour les exemples 1 à 4 ci-dessus. | | | | |

Au moment de l'emploi, on a mélangé poids pour poids chacune des compositions tinctoriales ci-dessus avec une solution aqueuse à 6.10-3 mol% de persulfate d'ammonium.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **9** | 10 ± 0,2 | Acajou irisé |
| **10** | 10 ± 0,2 | Blond naturel cendré |
| **11** | 10 ± 0,2 | Blond clair naturel cendré |
| **12** | 10 ± 0,2 | Châtain clair naturel cendré mat |

### EXEMPLES 13 à 16 DE TEINTURE EN MILIEU NEUTRE

On a préparé les compositions tinctoriales conformes à l'invention suivantes (teneurs en grammes) :

| **EXEMPLE** | **13** | **14** | **15** | **16** |
|---|---|---|---|---|
| Monochlorure de 3-[3-(2-aminophénylamino)-propyl]-1 -méthyl-3H-imidazol-1-ium, chlorhydrate (composé de formule (l)) | 0,909 | 0,909 | 0,909 | 0,909 |
| Méta-aminophénol (coupleur) | 0,327 | - | - | - |
| 4,5-diamino 1-éthyl 3-méthyl pyrazole 2HCl (base d'oxydation) | - | 0,639 | - | - |
| Dichlorhydrate de 2,4-diamino phénoxyéthanol (coupleur) | - | - | - | 0,723 |
| Support de teinture commun n°2 | (**) | (**) | (**) | (**) |
| Eau déminéralisée q.s.p. | 100 g | 100 g | 100 g | 100 g |

| | | | | |
|---|---|---|---|---|
| (**) Support de teinture commun n°2 : | | | | |

II est identique à celui utilisé pour les exemples 5 à 8 ci-dessus.

Au moment de l'emploi, on a mélangé poids pour poids chacune des compositions tinctoriales ci-dessus avec une solution aqueuse à 6.10-3 mol% de persulfate d'ammonium.

Le mélange obtenu a été appliqué sur des mèches de cheveux gris permanentés à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **13** | 5,7 ± 0,2 | Blond cendré mat |
| **14** | 5,7 ± 0,2 | Blond doré cuivré |
| **15** | 5,7 ± 0,2 | Blond foncé cuivré doré |
| **16** | 5,7 ± 0,2 | Blond doré acajou |

### EXEMPLE 17 DE TEINTURE A L'AIR

On a préparé, au moment de l'emploi, la composition tinctoriale conforme à l'invention suivante :
- Monochlorure de 3-[3-(2-amino-phénylamino)-propyl]1-méthyl-3H-imidazol-1-ium, chlorhydrate (composé de formule (l)) 1,818 g
- Ethanol à 96° 20 g
- Tampon pH 9,5 NH₄OH/NH₄Cl (1M/1M) 10 g
- Eau déminéralisée qsp 100 g

Cette composition a été appliquée sur des mèches de cheveux gris permanentés à 90% de blancs, et on laissé la coloration se développer pendant 30 minutes, sans ajout d'agent oxydant autre que l'oxygène de l'air.

Les cheveux ont ensuite été rincés, lavés avec un shampooing standard, rincés à nouveau puis séchés.

Les cheveux ont été teints dans une nuance cuivré doré.

## Revendications

1. Composés de formule (I) suivante, et leurs sels d'addition avec un acide : 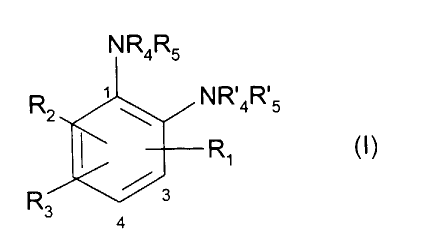 dans laquelle :
• R₁, R₂, R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆); un radical carboxy ; un radical alkyl(C₁-C₆) carboxy ; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle; un radical aminosulfonylalkyle(C₁-C₆); un radical N-Z-aminosulfonylalkyle(C₁-C₆); un radical N-alkyl(C₁-C₆)aminosulfonylalkyle(C₁-C₆) ; un radical N,N-dialkyl(C₁-C₆)aminosulfonylalkyle(C₁-C₆); un radical carbamyle ; un radical N-alkyl(C₁-C₆)carbamyle; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z dans lequel le bras de liaison D comporte une fonction cétone directement rattachée à l'atome d'azote dudit groupe amino ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et le groupement Z ;
• R₄, R'₄, R₅ et R'₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-Z-aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
• Z est choisi parmi les groupements cationiques insaturés de formules (II) et (III) suivantes, et les groupements cationiques saturés de formule (lV) suivante : 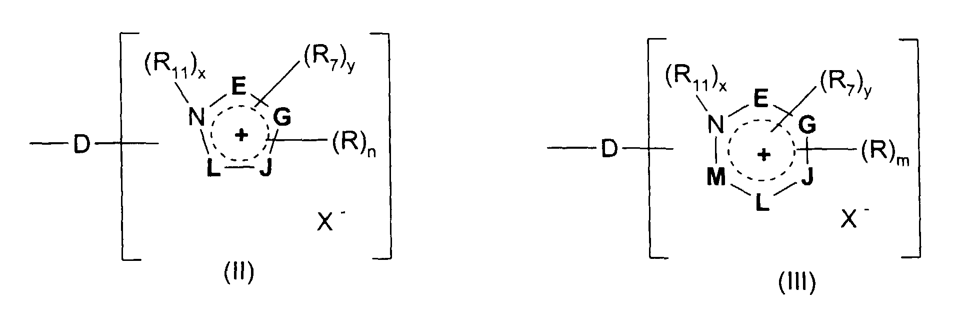 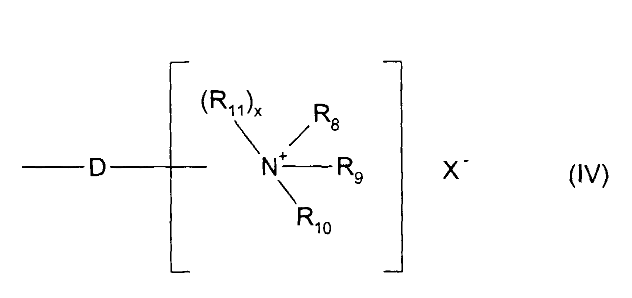 dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
• les sommets E, G, J, L et M, identiques ou différents, représentent un atome de carbone, d'oxygène, de soufre ou d'azote ;
• n est un nombre entier compris entre 0 et 4 inclusivement ;
• m est un nombre entier compris entre 0 et 5 inclusivement ;
• les radicaux R, identiques ou différents, représentent un second groupement Z identique ou différent du premier groupement Z, un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente un radical alkyle en C₁-C₆, un radical monohydroxyalkyle en C₁-C₆, un radical polyhydroxyalkyle en C₂-C₆, un radical cyanoalkyle en C₁-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle, un second groupement Z identique ou différent du premier groupement Z ;
• R₈, R₉ et R₁₀, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical amidoalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₈, R₉ et R₁₀ peut également représenter un second groupement Z identique ou différent du premier groupement Z ;
• R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x et y sont des nombres entiers égaux à 0 ou 1 ; avec les conditions suivantes :
- dans les groupements cationiques insaturés de formule (ll) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que:
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements cationiques insaturés de formule (lll) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
- dans les groupements cationiques de formule (lV) :
- lorsque x = 0, alors le bras de liaison est rattaché à l'atome d'azote portant les radicaux R₈ à R₁₀,
- lorsque x = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X⁻ représente un anion monovalent ou divalent ;
étant entendu que :
- le nombre de groupements cationiques insaturés Z de formule (II) ou (III) est au moins égal à 1 ;
- lorsque R₄ et/ou R₅ et/ou R'₄ et/ou R'₅ représente un groupement Z dans lequel le bras de liaison D représente une chaîne alkyle comportant une fonction cétone, alors ladite fonction cétone n'est pas directement rattachée à l'atome d'azote du groupement -NR₄R₅ ou -NR'₄R'₅.

2. Composés selon la revendication 1, caractérisées par le fait que X⁻ représente un atome d'halogène tel que le chlore, le brome, le fluor ou l'iode, un hydroxyde, un hydrogènesulfate, ou un alkyl(C₁-C₆)sulfate.

3. Composés selon la revendication 1 ou 2, caractérisées par le fait que ledit cycle saturé à 5 ou 6 chaînons carboné ou contenant un ou plusieurs hétéroatomes est un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle.

4. Composés selon l'une quelconque des revendications précédentes, caractérisées par le fait les cycles des groupements insaturés Z de formule (ll) sont choisis parmi les cycles pyrrolique, imidazolique, pyrazolique, oxazolique, thiazolique et triazolique.

5. Composés selon l'une quelconque des revendications précédentes, caractérisées par le fait les cycles des groupements insaturés Z de formule (III) sont choisis parmi les cycles pyridinique, pyrimidinique, pyrazinique, oxazinique et triazinique.

6. Composés selon l'une quelconque des revendications précédentes, caractérisés par le fait qu'ils sont choisis parmi :
- le monochlorure de 3-[3-(2-amino-phénylamino)-propyl]-1-méthyl-3H-imidazol-1-ium ;
- le monochlorure de 3-[2-(2-amino-phénylamino)-éthyl]-1-méthyl-3H-imidazol-1-ium ;
- le dichlorure de 4-[2-(1-méthyl-3H-imidazol-1-ium)-éthoxy]-N2-[2-(1-méthyl-3H-imidazol-1-ium)-éthyl]-benzène-1,2-diamine ;
- le monochlorure de 3-[2-(2-amino-4-méthyl-phénylamino)-éthyl]-1-éthyl-3H-imidazol-1-ium ;
- le dichlorure de 3-[3-(2-amino-phénylamino)-propyl]-1-(3-triméthyl-ammonium-2-hydroxy-propyl)-3H-imidazol-1-ium ;
- le monobromure de 3-[3-(2-amino-phénylamino)-propyl]-1-(2-hydroxy-éthyl)-3H-imidazol-1-ium
- le monochlorure de 3-([2-(2-amino-phénylamino)-éthylcarbamoyl]-méthyl)-1-méthyl-3H-imidazol-1-ium ;
- le monochlorure de 1-[2-(2-amino-4-chloro-phénylamino)-éthyl]-pyridinium ;
- le monochlorure de 3-[2-(2-amino-5-méthoxy-phénylamino)-éthyl]-1-méthyl-3H-imidazol-1-ium ;
- le monochlorure de 3-[2-(2-amino-5-méthylsulfanyl-phénylamino)-éthyl]-1-méthyl-3H-imidazol-1-ium ;
et leurs sels d'addition avec un acide.

7. Composés selon l'une quelconque des revendications précédentes, caractérisés par le fait que les sels d'addition avec un acide sont choisis parmi les chlorhydrates, les bromhydrates, les sulfates, les citrates, les succinates, les tartrates, les lactates et les acétates.

8. Utilisation des composés de formule (I) tels que définis à l'une quelconque des revendications 1 à 7, à titre de base d'oxydation, de coupleur ou de colorant auto-oxydable pour la teinture d'oxydation des fibres kératiniques, et en particulier des fibres kératiniques humaines telles que les cheveux.

9. Composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) tel que défini à l'une quelconque des revendications 1 à 7.

10. Composition selon la revendication 9, caractérisée par le fait que le ou les composés de formule (I) représentent de 0,0005 à 12 % en poids du poids total de la composition tinctoriale.

11. Composition selon la revendication 9, caractérisée par le fait que le ou les composés de formule (I) représentent de 0,005 à 6 % en poids du poids total de la composition tinctoriale.

12. Composition selon l'une quelconque des revendications 9 à 11, caractérisée par le fait qu'elle renferme une ou plusieurs bases d'oxydation choisie parmi les paraphénylènediamines, les bis-phénylalkylènediamines, les para-aminophénols, les ortho-aminophénols et les bases hétérocycliques.

13. Composition selon la revendication 12, caractérisée par le fait que les paraphénylènediamines sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl) amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

14. Composition selon la revendication 12, caractérisée par le fait que les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

15. Composition selon la revendication 12, caractérisée par le fait que les paraaminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

16. Composition selon la revendication 12, caractérisée par le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

17. Composition selon la revendication 12, caractérisée par le fait que les bases hétérocycliques sont choisies parmi les dérivés pyridiniques, les dérivés pyrimidiniques et les dérivés pyrazoliques.

18. Composition selon l'une quelconque des revendications 12 à 17, caractérisée par le fait que la ou les bases d'oxydation représentent de 0,0005 à 12 % en poids du poids total de la composition tinctoriale.

19. Composition selon la revendication 18, caractérisée par le fait que la ou les bases d'oxydation représentent de 0,005 à 6 % en poids du poids total de la composition tinctoriale.

20. Composition selon l'une quelconque des revendications 9 à 19, caractérisée par le fait qu'elle renferme un ou plusieurs coupleurs choisis parmi les métaphénylénediamines, les méta-aminophénols, les métadiphénols et les coupleurs hétérocycliques.

21. Composition selon la revendication 20, caractérisée par le fait que les coupleurs sont choisis parmi le 2-méthyl 5-amino phénol, le 5-N-(β-hydroxyéthyl) amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1 -(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

22. Composition selon la revendication 20 ou 21, caractérisée par le fait que le ou les coupleurs représentent de 0,0001 à 10 % en poids environ du poids total de la composition tinctoriale.

23. Composition selon la revendication 22, caractérisée par le fait que le ou les coupleurs représentent de 0,005 à 5 % en poids environ du poids total de la composition tinctoriale.

24. Composition selon l'une quelconque des revendications 9 à 23, caractérisée par le fait que les sels d'addition avec un acide sont choisis parmi les chlorhydrates, les bromhydrates, les sulfates, les citrates, les succinates, les tartrates, les lactates et les acétates.

25. Procédé de teinture d'oxydation des fibres kératiniques et en particulier des fibres kératiniques humaines telles que les cheveux, caractérisé par le fait qu'on applique sur les fibres au moins une composition tinctoriale telle que définie à l'une quelconque des revendications 9 à 24, pendant un temps suffisant pour développer la coloration désirée, soit à l'air, soit à l'aide d'un agent oxydant.

26. Procédé selon la revendication 25, caractérisé par le fait que la coloration des fibres peut être effectuée sans addition d'un agent oxydant, au seul contact de l'oxygène de l'air.

27. Procédé selon la revendication 25, caractérisé par le fait qu'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

28. Procédé selon la revendication 27, caractérisé par le fait que l'agent oxydant est choisi parmi le peroxyde d'hydrogène, le peroxyde d'urée, les bromates de métaux alcalins, les persels tels que les perborates et persulfates et les enzymes telles que les peroxydases et les oxydo-réductases à 2 électrons.

29. Dispositif à plusieurs compartiments, ou "kit" de teinture à plusieurs compartiments, dont un premier compartiment renferme une composition tinctoriale telle que définie à l'une quelconque des revendications 9 à 24 et un second compartiment renferme une composition oxydante.
